# FASCICULE DE BREVET EUROPEEN

(11) **EP 2 212 095 B1**
(45) Date de publication et mention de la délivrance du brevet: **18.01.2012**
(21) Numéro de dépôt: 08857219.3
(22) Date de dépôt: 18.11.2008
(51) Int. Cl.: B29C 49/42, B29C 49/78, B29C 49/06

(54) **Installation et procédé de convoyage de préformes**
Vorrichtung und Verfahren zum Transport von Vorformlingen
Apparatus and method for conveying preforms

(30) Priorité: 19.11.2007 FR 0708110
(43) Date de publication de la demande: 04.08.2010
(60) Demande divisionnaire: 11189032.3
(73) Titulaire: Sidel Participations, 76930 Octeville Sur Mer (FR)
(72) Inventeur: MIE, Patrick, F-76930 Octeville-sur-Mer (FR)
(74) Mandataire: Cabinet Plasseraud
(86) Numéro de dépôt international: PCT/FR2008/052074
(87) Numéro de publication internationale: WO 2009/071792

(56) Documents cités:
- FR-A- 2 789 932
- FR-A- 2 872 805
- FR-A- 2 890 061

## Description

L'invention se rapporte de manière générale au domaine de fabrication de récipients en matériau thermoplastique obtenus après chauffage et soufflage d'un corps creux appelé communément préforme, et elle porte plus particulièrement sur un système de convoyage comportant une série de dispositifs de transport qui sont munis chacun d'au moins un organe de préhension destiné à porter une préforme en vue de la faire circuler en position verticale à l'intérieur de l'installation.

Ainsi, l'invention s'applique aux installations de traitement en ligne pour la fabrication ou le traitement de préformes en matière thermoplastique telle que du PET, dans lesquelles installations lesdites préformes sont déplacées individuellement les unes à la suite des autres à l'aide de dispositifs de transport aptes à les maintenir par leur col, de manière à laisser leur corps dégagé en vue du chauffage.

De manière courante, une préforme se présente sous la forme générale d'un corps cylindrique tubulaire fermé à l'une de ses extrémités axiales et prolongé à son autre extrémité ouverte par un col présentant déjà la forme définitive du col du récipient final ainsi qu'une collerette s'étendant sensiblement radialement. Dans la suite de la description, il est entendu par « récipient pourvu d'un col », tout corps creux présentant un col, à savoir des préformes, à partir desquelles sont fabriqués des récipients par soufflage ou étirage-soufflage, c'est-à-dire des récipients ayant leur forme définitive et devant subir un traitement additionnel (tel qu'une étape d'étiquetage ou de remplissage).

Dans le cas particulier du transport de préformes, en vue de leur mise en température par chauffage préalablement à l'étape de soufflage, il est nécessaire de prévoir un dispositif de transfert permettant de prendre chaque préforme par son col (« vêtissage ») pour la déplacer dans une zone de chauffage, en général dans un four tunnel de conditionnement thermique, dans laquelle zone le corps de ladite préforme doit être chauffé à la température de transition vitreuse de la matière thermoplastique, tout en restant à une température inférieure à la température de cristallisation.

Pour réaliser ce transfert, il est connu d'utiliser des dispositifs de transport munis d'organes de préhension qui comportent un mandrin propre à être engagé à l'intérieur du col de la préforme (vêtissage intérieur) et qui maintient cette dernière simplement par adhérence du fait de la présence des forces de pression et de frottement.

Il est présenté, dans le document FR 2 706 876, au nom de la Demanderesse, un dispositif de vêtissage intérieur de préformes à partir d'une tête de préhension ayant une extrémité à bague fendue.

Selon le même principe du vêtissage intérieur, il est connu, comme présenté dans le document FR 2 794 109 au nom de la Demanderesse, de réaliser un système de convoyage de préformes formé d'un ensemble d'éléments de transport comprenant chacun un dispositif de préhension muni à son extrémité d'une bague annulaire fendue formée de plusieurs secteurs ; lorsque la bague est engagée intérieurement dans le col d'une préforme, les secteurs qui sont sollicités radialement vers l'extérieur, par des moyens élastiques, sont aptes à prendre appui sur la surface intérieure cylindrique du col.

De manière alternative, il est également possible que la préforme soit saisie au niveau de la face externe de son col (vêtissage extérieur), comme décrit, par exemple, dans le document FR 2 882 963 au nom de la Demanderesse.

Lors du passage de cette préforme dans le four de conditionnement thermique, la préforme subit une rotation sur elle-même afin de permettre un chauffage homogène sur toute la surface extérieure de la préforme. Or, lors du vêtissage d'une préforme, aussi bien intérieur qu'extérieur, il est possible que celle-ci ne soit pas axialement alignée avec la tête de préhension.

Dans ce cas, du fait du désaxage de la préforme et de son mouvement de rotation sur elle-même, le corps de la préforme, et en particulier son extrémité, est susceptible de venir en contact avec les lampes de chauffage du four et de causer des dégâts importants en les cassant, et aussi, parfois, la préforme peut s'enflammer.

Ainsi, afin de prévenir ces incidents, comme la détérioration des lampes de chauffage du four, il est nécessaire de prévoir, avant leur entrée dans le four, une opération de dévêtissage des préformes, c'est-à-dire une opération qui consiste à éjecter toutes les préformes qui sont susceptibles de générer des incidents lors de leur passage dans le four de chauffage.

Cette opération de dévêtissage permet, d'une manière générale, d'éviter de chauffer des préformes qui présenteraient un défaut de conception en entrée du four.

Le document FR 2 872 805 au nom de la Demanderesse, décrit un système de dévêtissage de préformes.

Des moyens de détection optique sont prévus pour déterminer quelles sont les préformes à dévêtir et, sous l'effet d'un appareil de commande approprié, du genre automate, un organe d'éjection intervient pour dégager les préformes en question, qui tombent alors dans un bac de récupération, par exemple.

Ce système, bien que donnant entière satisfaction, est néanmoins complexe à mettre en oeuvre et, surtout, il bride les cadences de production de l'installation ; en effet, le temps de réponse de l'organe d'éjection, en vue du dévêtissage, est relativement long.

De plus, du fait des cadences de production actuelle et donc de la vitesse de défilement des préformes, il n'est pas possible d'éjecter une seule préforme car, du fait du temps de réponse des moyens pneumatiques de commande, ce sont plusieurs préformes qui sont touchées par l'organe d'éjection et, en général, de manière courante, ce sont trois préformes successives qui, en moyenne, sont éjectées.

Du fait du coût de fabrication des préformes, et afin d'empêcher la mise au rebut de préformes correctement placées sur la tête de vêtissage ou ne présentant aucun défaut, il serait particulièrement intéressant de réaliser un dispositif de dévêtissage de préformes permettant le dévêtissage uniquement de la préforme mal solidarisée ou mal formée ; ledit dispositif pouvant être mis en oeuvre à des cadences élevées de circulation des préformes. Il serait également particulièrement intéressant de réaliser un système de dévêtissage pouvant également s'appliquer au dévêtissage des préformes mais et aussi à tout type de corps creux.

De plus, du fait de ce dévêtissage de plusieurs préformes disposées les unes à côté des autres, il existe des espaces libres importants dans la chaîne de convoyage des préformes (trois têtes de vêtissage successives). Ces grands espaces entre les préformes, dans le four, perturbent la chauffe des autres préformes lors de leur passage devant les lampes dudit four ; il en résulte des défauts d'homogénéité du chauffage des préformes.

Par ailleurs, une évolution actuelle dans le cadre de la fabrication des préformes, tend à diminuer les dimensions de la collerette, laquelle devient de moins en moins épaisse et de moins en moins large, d'où des problèmes de vêtissage plus fréquents.

Ainsi, il serait également particulièrement avantageux de réaliser un système de dévêtissage qui ne soit pas dépendant de la taille d'une collerette prévue sur le col du corps creux.

La présente invention propose donc de résoudre les différents problèmes liés à l'art antérieur à l'aide d'un système de dévêtissage de corps creux permettant de dévêtir une seule préforme à des cadences de convoyage élevées, lequel système, en plus, ne dépend pas des caractéristiques dimensionnelles de la collerette de ladite préforme.

Selon un premier de ses aspects, la présente invention concerne une installation de convoyage de préformes vers une unité de traitement du genre four de chauffe dans lequel elles sont portées à une température appropriée pour être ensuite mise en forme par étirage-soufflage, selon la revendication 1.

Toujours selon l'invention, les moyens susceptibles d'appliquer une pression dans la préforme sélectionnée, sur son fond, sont constitués :
- d'une buse, capable de projeter un jet de fluide sous pression vers le fond de ladite préforme sélectionnée, et,
- d'un orifice en forme d'alésage aménagé dans l'organe et dans la tête de préhension de ladite préforme sélectionnée, lequel jet de fluide sous pression étant administré lors du passage dudit alésage devant ladite buse.

Selon une autre disposition de l'invention, l'alésage dans lequel est injecté le fluide sous pression destiné à la préforme sélectionnée comporte une ouverture amont en forme d'entonnoir qui présente un rétrécissement de sa section transversale en direction de l'ouverture aval.

Toujours selon l'invention, l'automate utilisé pour la commande de l'injection du fluide sous pression comporte un programme aménagé pour établir une phase appropriée pour l'administration dudit fluide sous pression vers la préforme sélectionnée, laquelle phase s'étend sur une durée sensiblement supérieure au temps mis par ladite préforme sélectionnée pour passer devant la buse de projection de façon à garantir un temps d'injection dudit fluide sous pression dans l'alésage qui est suffisamment long pour assurer l'éjection de ladite préforme sélectionnée.

L'invention concerne également le procédé de dévêtissage de préformes pendant leur convoyage vers le four de chauffe, pour éliminer chaque préforme qui est susceptible de créer un incident lors de son passage dans ledit four de chauffe, selon la revendication 6.

Toujours selon l'invention, le procédé de dévêtissage consiste à séparer la préforme indésirable de sa tête de préhension par le biais d'une injection, dans un alésage approprié de ladite tête de préhension, d'un jet de fluide sous pression, qui pénètre dans le volume intérieur de ladite préforme indésirable.

Selon une autre disposition de l'invention, le procédé de dévêtissage consiste à anticiper le passage de la préforme indésirable pour déclencher l'injection du fluide sous pression dans ladite préforme et il consiste aussi à maintenir cette injection dudit fluide sous pression au moins jusqu'au passage complet de la tête de préhension devant la buse de projection dudit fluide.

La présente invention est maintenant décrite à l'aide d'un exemple uniquement illustratif et nullement limitatif de la portée de la présente invention, et à partir de la figure suivante qui représente une vue en coupe axiale d'un dispositif de transport et de vêtissage d'un corps creux selon l'invention, lequel dispositif, en coopération avec une buse de soufflage, permet d'effectuer une opération de dévêtissage des préformes.

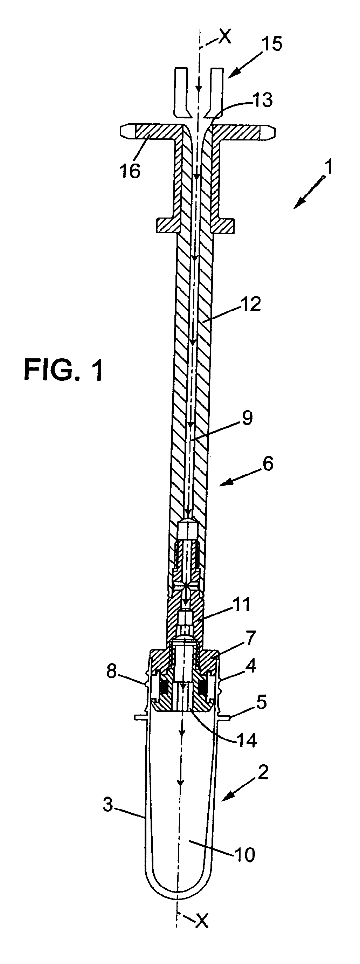

De manière simplifiée, la figure 1 représente une vue en coupe axiale d'un dispositif 1 de transport d'un corps creux, du type préforme 2 en matière thermoplastique. La préforme 2 présente un corps 3 s'étendant de manière sensiblement longitudinale, et un col 4 séparé du corps 3 par une collerette 5 s'étendant selon une direction sensiblement transversale.

Le dispositif 1 de transport comprend un organe 6 de préhension avec une tête 7 de préhension propre à être solidarisée avec la préforme 2, et préférentiellement, à être solidarisé de manière étanche avec son col 4.

Il est représenté sur la figure 1 un vêtissage dit « intérieur » de la préforme 2 et du type tel que décrit dans le document FR 2 794 109 précité, c'est-à-dire que la tête 7 de préhension est insérée à l'intérieur du col 4 et prend donc appui sur la face interne du col 4.

Toutefois, il convient de noter que le principe selon l'invention s'applique également à un vêtissage dit « extérieur » de la préforme 2, c'est-à-dire quand la tête 7 de préhension prend appui sur la face externe du col 4, en général en prenant appui sur le filetage 8 du col 4.

Selon l'invention, l'organe 6 de préhension présente au moins un alésage 9 débouchant à travers la tête 7 de préhension.

Ainsi, selon une première application de l'invention, la préforme 2 est apte à être désolidarisée de la tête 7 de préhension par injection d'un fluide sous-pression (préférentiellement de l'air comprimé sous une pression de l'ordre d'au moins plusieurs bars, de 2 à 5 bars) dans l'alésage 9 débouchant alors dans le volume 10 fermé qui est formé par le corps 3 de la préforme 2 solidarisée à ladite tête 7 et ce, que le volume 10 soit parfaitement étanche ou non.

De manière alternative, l'intérieur de la préforme 2 peut être aseptisé par injection d'un fluide aseptique dans l'alésage 9 débouchant dans le volume 10 fermé qui est formé par le corps 3 creux solidarisé à ladite tête 7.

De manière générale, il est possible d'injecter tout type de fluide à travers l'alésage 9 présentant une interaction appropriée et/ou une action de traitement souhaitée sur le volume 10 fermé qui est formé par le corps 3 creux solidarisé à la tête 7.

L'alésage 9 est donc prévu traversant l'organe 6 de préhension, de manière sensiblement rectiligne à l'intérieur dudit organe 6. Cet alésage 9 est de forme générale cylindrique et il est formé centralement dans l'organe 6, c'est-à-dire qu'en considérant que l'organe 6 de préhension définit un axe X-X principal de préhension, axe X-X par rapport auquel il est déterminé si la préforme 2 est correctement solidarisée à la tête 7 de préhension et alignée avec celle-ci, alors l'alésage 9 est sensiblement formé de manière coaxiale à l'axe X-X principal de préhension.

De manière plus précise, l'organe 6 de préhension comprend un mandrin 11 présentant ladite tête 7 de préhension ainsi qu'une tige 12 de préhension sur laquelle est fixé, préférentiellement de manière amovible, ledit mandrin 11.

L'alésage 9 est donc formé et creusé aussi bien dans la tige 12 de préhension que dans le mandrin 11.

Afin de limiter les pertes de charges lors de l'injection du fluide sous pression dans l'alésage, ledit alésage est prévu sensiblement rectiligne à l'intérieur de l'organe de préhension et il est, de manière avantageuse, de forme générale sensiblement cylindrique.

Plus précisément, l'alésage 9 présente une ouverture 13 amont pour l'injection d'un fluide ainsi qu'une ouverture 14 aval dans la tête 7 de préhension. L'ouverture 13 amont présente un rétrécissement de sa section transversale en direction de l'ouverture 14 aval.

Au niveau de l'ouverture 13 amont, afin de permettre à une quantité maximale de fluide d'être canalisée, dirigée et d'entrer dans l'alésage 9, il est donc prévu que l'ouverture 13 amont présente une section transversale plus grande que la section transversale de la majeure partie de l'alésage 9, et notamment plus grande que la section transversale de l'ouverture 14 aval.

Même s'il est représenté sur la figure 1 un dispositif 1 de transport avec un seul alésage 9 central, il est également possible de prévoir plusieurs alésages 9, par exemple disposés concentriquement à l'axe X-X principal de préhension, ou alors de prévoir un alésage 9 se divisant en plusieurs alésages secondaires tous débouchant dans la tête 7 de préhension.

La présente invention porte également, selon un second de ses aspects, et de manière plus générale, sur une installation de convoyage de corps 2 creux comprenant une pluralité de dispositifs 1 de transport qui circulent alignés les uns à la suite des autres sur une chaîne sans fin, non représentée, de type connu en soi, comme visible, par exemple, sur le document FR 2 872 805 précité.

L'installation de convoyage comprend des moyens d'injection d'un jet de fluide, tel qu'un fluide de soufflage sous-pression, par exemple à une pression comprise entre 2 et 5 bars, sachant que l'on trouve, sur ce type de machine, une pression qui est disponible jusqu'à 40 bars, lesquels moyens d'injection du fluide de soufflage sont dirigés vers un point de passage d'au moins un alésage 9 formé intérieurement dans un dispositif 1 de transport lors de sa circulation sur la chaîne sans fin.

De manière préférentielle, les moyens d'injection se présentent sous la forme d'une buse 15 de soufflage qui est fixe, solidaire du châssis de l'installation, non représenté, laquelle buse 15 est donc dirigée vers un point de passage de l'ouverture 13 amont de l'alésage 9 lors de la circulation des dispositifs 1 de transport associés à la chaîne sans fin.

Afin d'éviter une perte trop importante du fluide injecté par la buse 15, ladite buse 15 est située en regard d'un point de passage de l'ouverture 13 amont de l'alésage des dispositifs 1 de transport et elle n'est écartée que d'une très faible distance par rapport à cette ouverture 13 amont, par exemple de l'ordre du millimètre, voire quelques millimètres.

Selon une application de l'invention, l'installation de convoyage peut comprendre également des moyens de détection du genre caméra de reconnaissance de forme (non représentée sur la figure mais de type connu en soi), pour déceler un défaut d'alignement du corps 3 de la préforme 2 avec l'axe X-X principal de préhension et l'axe de la tête 7 de préhension, ce grâce à quoi il est déterminé si la préforme 2 transportée par ladite tête 7 de préhension doit être désolidarisée de cette dernière par l'injection du fluide de soufflage sous-pression.

La commande de cette injection peut être réalisée par le biais d'un automate qui reçoit et traite les informations provenant des moyens de détection dont il était question auparavant.

Cet automate comporte un programme aménagé pour organiser la commande de l'injection du fluide sous pression dans la préforme reconnue comme présentant un défaut susceptible de générer des incidents dans le four de chauffage de ces préformes.

L'aménagement du programme d'injection comporte une phase appropriée pour l'administration du fluide sous pression vers la préforme sélectionnée, laquelle phase s'étend, par exemple, sur une durée sensiblement supérieure au temps mis par ladite préforme sélectionnée pour passer devant la buse de projection de façon à garantir un temps d'injection dudit fluide sous pression dans l'alésage qui soit suffisamment long pour assurer l'éjection de ladite préforme sélectionnée.

Cette phase d'injection est également aménagée pour permettre une anticipation du déclenchement de l'injection du fluide sous pression dans ladite préforme indésirable face au passage de ladite préforme indésirable devant la buse 15 de projection dudit fluide et pour maintenir cette injection dudit fluide sous pression au moins jusqu'au passage complet de la tête 7 de préhension devant ladite buse 15 de projection dudit fluide.

L'invention permet donc, de manière générale, de réaliser un procédé de convoyage d'une préforme 2 solidarisée à un dispositif 1 de transport, procédé comprenant une étape d'injection d'un fluide à travers au moins un alésage 9 prévu traversant ledit dispositif 1 de transport et débouchant dans le volume 10 intérieur du corps 3 de la préforme 2 solidarisée audit dispositif 1 de transport.

Selon une autre forme d'application de l'invention, il est injecté un fluide aseptique à travers l'alésage 9. Alternativement, il est injecté un fluide sous-pression, ce qui permet de désolidariser le corps 2 creux de la tête 7 de vêtissage.

Ainsi, de manière résumée, selon une application de l'invention, il est détecté, à l'aide des moyens de détection, si une préforme 2 est désaxée de l'axe X-X ou présente un défaut de fabrication. Il est alors déterminé, à l'aide de l'automate et de programmes appropriés et/ou de moyens de calcul, quand le dispositif 1 de transport avec cette préforme, et plus précisément son alésage 9, va passer en regard des moyens 15 d'injection de fluide de soufflage, pour ensuite commander l'injection de ce fluide pour dévêtir la préforme (ou n'importe quel corps creux solidarisé sur une tête 7 de vêtissage).

Le temps de réponse des moyens d'injection de fluide est nettement plus rapide que les temps de réponse des moyens pneumatiques à piston selon l'art antérieur précité, ce qui permet de ne dévêtir qu'une seule préforme à la fois.

Ainsi, selon une première application de l'invention, le corps creux est apte à être désolidarisé de la tête de préhension par injection d'un fluide de soufflage sous-pression dans l'alésage débouchant alors dans le volume fermé qui est formé par le corps creux solidarisé à ladite tête.

De manière alternative, l'intérieur du corps creux peut être aseptisé par injection d'un fluide aseptique dans l'alésage débouchant dans le volume fermé qui est formé par le corps creux solidarisé à ladite tête.

De manière plus générale, l'invention peut trouver sa place sur une installation de transport de récipients à col, où il est parfois nécessaire de dévêtir les corps creux présentant un défaut, comme, par exemple, en sortie de l'unité de soufflage des préformes.

## Revendications

1. Installation de convoyage de préformes (2) vers une unité de traitement du genre four de chauffe dans lequel elles sont portées à une température appropriée pour être ensuite mises en forme par étirage-soufflage, laquelle installation comporte :
- des organes (6) de préhension portés par une chaîne sans fin de convoyage et qui sont munis, chacun, d'une tête (7) de préhension pour porter lesdites préformes (2) et pour les transférer dans ledit four de chauffe,
- au moins un système de détection disposé sur le parcours desdites préformes (2), en amont dudit four de chauffage, lequel système de détection, du genre caméra de reconnaissance de formes, sélectionne les préformes susceptibles de provoquer des incidents dans ledit four de chauffe, comme, par exemple, des préformes dont l'alignement ne correspond pas avec celui de l'axe principal de leur tête (7) de préhension,
- des moyens pour désolidariser chaque préforme sélectionnée de sa tête (7) de préhension,
- un appareil, du genre automate, pour commander l'éjection de la préforme sélectionnée en fonction des informations en provenance dudit système de détection,
- des moyens de récupération des préformes éjectées,
**caractérisée en ce qu'**elle comprend, des moyens pour appliquer une pression à l'intérieur de la préforme (2) sélectionnée, sur son fond, et spécialement une pression suffisante pour vaincre les forces d'adhérence entre ladite préforme sélectionnée et sa tête (7) de préhension, afin de les désolidariser.

2. Installation selon la revendication 1, **caractérisée en ce que** les moyens susceptibles d'appliquer une pression dans la préforme sélectionnée, sur son fond, sont constitués :
- d'une buse (15), capable de projeter un jet de fluide sous pression vers le fond de ladite préforme sélectionnée, et,
- d'un orifice en forme d'alésage (9) aménagé dans l'organe (6) et dans la tête (7) de préhension de ladite préforme sélectionnée, ledit jet de fluide sous pression étant administré lors du passage dudit alésage (9) devant ladite buse (15).

3. Installation selon la revendication 2, **caractérisée en ce que** l'alésage (9), dans lequel est injecté le fluide sous pression destiné à la préforme sélectionnée, comporte une ouverture (13) amont en forme d'entonnoir qui présente un rétrécissement de sa section transversale en direction de l'ouverture (14) aval.

4. Installation selon la revendication 3, **caractérisée en ce qu'**elle comporte des moyens d'injection du fluide sous une pression allant de 2 à 5 bars.

5. Installation selon la revendication 1, **caractérisée en ce que** l'automate utilisé pour la commande de l'injection du fluide sous pression comporte un programme aménagé pour établir une phase appropriée pour l'administration dudit fluide sous pression vers la préforme sélectionnée, laquelle phase s'étend sur une durée sensiblement supérieure au temps mis par ladite préforme sélectionnée pour passer devant la buse (15) de façon à garantir un temps d'injection dudit fluide sous pression dans l'alésage (9) qui soit suffisamment long pour assurer l'éjection de ladite préforme sélectionnée.

6. Procédé de dévêtissage de préforme(s) pendant leur convoyage vers le four de chauffe, pour éliminer chaque préforme susceptible de créer un incident lors de son passage dans ledit four de chauffe, ledit procédé consistant :
- à surveiller les préformes (2) qui défilent, avant leur entrée dans ledit four de chauffe, pour détecter, au niveau desdites préformes, un vice susceptible de générer des incidents dans ledit four de chauffe,
- à désolidariser chaque préforme, qualifiée d'indésirable, de sa tête (7) de préhension, en amont dudit four de chauffe,
- à recueillir la préforme rejetée dans un conteneur approprié,
**caractérisé en ce qu'**il consiste à appliquer une pression appropriée à l'intérieur de ladite préforme indésirable, sur son fond, et spécialement une pression suffisante pour vaincre les forces de pression et de frottement qui s'exercent entre ladite préforme indésirable et sa tête (7) de préhension.

7. Procédé de dévêtissage de préforme(s) selon la revendication 6, **caractérisé en ce qu'**il consiste à séparer la préforme indésirable de sa tête (7) de préhension par le biais d'une injection, dans un alésage (9) approprié de ladite tête (7) de préhension, d'un jet de fluide sous pression qui pénètre dans le volume intérieur de ladite préforme indésirable.

8. Procédé de dévêtissage de préforme(s) selon la revendication 6, **caractérisé en ce qu'**il consiste à injecter le jet de fluide sous une pression allant de 2 à 5 bars.

9. Procédé de dévêtissage de préforme(s) selon la revendication 6, **caractérisé en ce qu'**il consiste à anticiper le passage de la préforme indésirable pour déclencher l'injection du jet de fluide sous pression dans ladite préforme et à maintenir cette injection dudit jet de fluide au moins jusqu'au passage complet de la tête (7) de préhension devant la buse (15) de projection dudit fluide.

## Claims

1. An installation for conveying preforms (2) to a processing unit such as a heating oven in which they are taken to an appropriate temperature in order subsequently to be formed by stretch-blow molding, which installation comprises:
- gripping members (6) which are carried by an endless conveyor chain and which are each furnished with a gripping head (7) in order to carry said preforms (2) and in order to transfer them into said heating oven,
- at least one detection system placed on the path of said preforms (2) upstream of said heating oven, which detection system, such as a shape-recognition camera, selects the preforms that are likely to cause incidents in said heating oven, such as, for example, preforms the alignment of which does not correspond with that of the main axis of their gripping head (7),
- means for detaching each selected preform from its gripping head (7),
- an apparatus, such as a controller, to command the ejection of the selected preform according to the information originating from said detection system,
- means for retrieving the ejected preforms, **characterized in that** it comprises means for applying a pressure to the inside of the selected preform (2), on its bottom, and in particular a sufficient pressure to overcome the forces of adhesion between said selected preform and its gripping head (7), in order to detach them.

2. The installation as claimed in claim 1, **characterized in that** the means capable of applying a pressure in the selected preform, on its bottom, consist:
- of a nozzle (15) capable of spraying a jet of pressurized fluid at the bottom of said selected preform, and,
- of an orifice in the shape of a bore (9) arranged in the member (6) and in the gripping head (7) of said selected preform, said jet of pressurized fluid being administered when said bore (9) passes in front of said nozzle (15).

3. The installation as claimed in claim 2, **characterized in that** the bore (9), into which the pressurized fluid intended for the selected preform is injected, comprises an upstream aperture (13) in the form of a funnel which has a narrowing of its cross section in the direction of the downstream aperture (14).

4. The installation as claimed in claim 3, **characterized in that** it comprises means for injecting fluid at a pressure of from 2 to 5 bar.

5. The installation as claimed in claim 1, **characterized in that** the controller used for commanding the injection of the pressurized fluid comprises a program arranged in order to establish an appropriate phase for the administration of said pressurized fluid to the selected preform, which phase extends for a period substantially greater than the time taken by said selected preform to pass in front of the nozzle (15) so as to ensure an injection time of said pressurized fluid into the bore (9) that is long enough to ensure the ejection of said selected preform.

6. A method for releasing a preform or preforms while they are conveyed to the heating oven, in order to eliminate each preform capable of creating an incident during its passage in said heating oven, said method consisting:
- in monitoring the preforms (2) which pass by, before they enter said heating oven, in order to detect, in said preforms, a flaw capable of causing incidents in said heating oven,
- in detaching each preform that is qualified as undesirable from its gripping head (7), upstream of said heating oven,
- in collecting the rejected preform in an appropriate container,
**characterized in that** it consists in applying an appropriate pressure to the inside of said undesirable preform, on its bottom, and in particular a sufficient pressure to overcome the forces of pressure and of friction that are applied between said undesirable preform and its gripping head (7).

7. The method for releasing a preform or preforms as claimed in claim 6, **characterized in that** it consists in separating the undesirable preform from its gripping head (7) via an injection, in an appropriate bore (9) of said gripping head (7), of a jet of pressurized fluid which penetrates the inner volume of said undesirable preform.

8. The method for releasing a preform or preforms as claimed in claim 6, **characterized in that** it consists in injecting the jet of fluid at a pressure of from 2 to 5 bar.

9. The method for releasing a preform or preforms as claimed in claim 6, **characterized in that** it consists in anticipating the passage of the undesirable preform in order to trigger the injection of the jet of pressurized fluid into said preform and in maintaining this injection of said jet of fluid at least until the gripping head (7) has entirely passed in front of the nozzle (15) for spraying said fluid.

## Patentansprüche

1. Vorrichtung zum Transport von Vorformlingen (2) in Richtung einer Heizofenbehandlungseinheit, in der sie auf eine Temperatur gebracht werden, die geeignet ist, um sie danach durch Streck-Blasziehen zu formen, wobei die Vorrichtung aufweist:
- Greifeinrichtungen (6), die durch eine endlose Förderkette getragen werden und die jeweils mit einem Greifkopf (7) zum Tragen der Vorformlinge (2) und zu ihrer Weitergabe in den Heizofen ausgestattet sind,
- mindestens ein Detektionssystem, das auf der Bahn der Vorformlinge (2) in Förderrichtung vor dem Heizofen angeordnet ist, wobei das Kamera-Formerkennungs-Detektionssystem Vorformlinge auswählt, die Zwischenfälle in dem Heizofen verursachen könnten, wie zum Beispiel Vorformlinge, deren Ausrichtung nicht mit der der Hauptachse ihres Greifkopfes (7) übereinstimmt,
- ein Mittel zum Trennen des jeweils ausgewählten Vorformlings von seinem Greifkopf (7),
- eine Schaltvorrichtung zum Steuern des Abwurfs des ausgewählten Vorformlings aufgrund der von dem Detektionssystem stammenden Informationen,
- ein Mittel zur Wiederverwertung der abgeworfenen Vorformlinge,
**dadurch gekennzeichnet, dass** sie ein Mittel zum Aufbringen eines Innendrucks auf den Boden des ausgewählten Vorformlings (2) aufweist, insbesondere einen zum Überwinden der Haftkräfte zwischen dem ausgewählten Vorformling und seinem Greifkopf (7) ausreichenden Druck, um diese zu trennen.

2. Vorrichtung gemäß Anspruch 1, **dadurch gekennzeichnet, dass** das Mittel zum Aufbringen eines Drucks auf den Boden des ausgewählten Vorformlings gebildet wird aus:
- einer Düse (15), die geeignet ist, einen unter Druck stehenden Fluidstrahl in Richtung des Bodens des ausgewählten Vorformlings zu spritzen und
- einer als Bohrung (9) ausgebildeten Öffnung, die in der Greifeinrichtung (6) und dem Greifkopf (7) des ausgewählten Vorformlings angeordnet ist, wobei der unter Druck stehende Fluidstrahl verabreicht wird, wenn die Bohrung (9) an der Düse (15) vorbeiläuft.

3. Vorrichtung gemäß Anspruch 2, **dadurch gekennzeichnet, dass** die Bohrung (9), in die das für den ausgewählten Vorformling bestimmte, unter Druck stehende Fluid eingespritzt wird, stromaufwärts eine trichterförmige Öffnung (13) aufweist, die eine Verengung ihres Querschnitts in Richtung der stromabwärts liegenden Öffnung (14) darstellt.

4. Vorrichtung gemäß Anspruch 3, **dadurch gekennzeichnet, dass** sie ein Mittel zum Einspritzen des Fluids unter einem Druck zwischen 2 und 5 bar aufweist.

5. Vorrichtung gemäß Anspruch 1, **dadurch gekennzeichnet, dass** die für die Steuerung des unter Druck stehenden Fluids verwendete Schaltvorrichtung ein Programm aufweist, das eingerichtet ist, eine zum Verabreichen des unter Druck stehenden Fluids in Richtung des ausgewählten Vorformlings geeignete Phase vorzusehen, wobei sich die Phase im Wesentlichen über eine längere Dauer erstreckt als die für den ausgewählten Vorformling vorgesehene Zeit, um vor der Düse (15) vorbeizulaufen, sodass eine Zeit des Einspritzens des unter Druck stehenden Fluids in die Bohrung (9) gewährleistet ist, die ausreichend lang ist, um den Abwurf des ausgewählten Vorformlings sicherzustellen.

6. Verfahren zum Ablösen eines Vorformlings/von Vorformlingen während ihres Transports zu dem Heizofen, um jeden Vorformling zu entfernen, der einen Zwischenfall während seines Durchgangs durch den Heizofen hervorrufen könnte, wobei das Verfahren aufweist:
- das Überwachen der vorbeilaufenden Vorformlinge (2) vor ihrem Eintritt in den Heizofen, um auf der Höhe der Vorformlinge einen Mangel zu detektieren, der Zwischenfälle in dem Heizofen erzeugen könnte,
- das Trennen eines jeden als unerwünscht gekennzeichneten Vorformlings von seinem Greifkopf in Förderrichtung gesehen vor dem Heizofen,
- das Einsammeln des abgeworfenen Vorformlings in einem geeigneten Behälter,
**dadurch gekennzeichnet, dass** es das gezielte Anwenden eines Drucks auf das Innere des unerwünschten Vorformlings umfasst, insbesondere eines Drucks, der zum Überwinden der Anpress- und Haftkräfte, die zwischen dem unerwünschten Vorformling und seinem Greifkopf (7) wirken, geeignet ist.

7. Verfahren zum Ablösen eines Vorformlings/von Vorformlingen gemäß Anspruch 6, **dadurch gekennzeichnet, dass** es das Trennen des unerwünschten Vorformlings von seinem Greifkopf (7) durch eine Einspritzung in eine geeignete Bohrung (9) des Greifkopfs (7) umfasst und zwar eines unter Druck stehenden Fluidstrahls, der in das innere Volumen des unerwünschten Vorformlings eindringt.

8. Verfahren zum Ablösen eines Vorformlings/von Vorformlingen gemäß Anspruch 6, **dadurch gekennzeichnet, dass** es das Einspritzen des Fluidstrahls unter einem Druck zwischen 2 und 5 bar umfasst.

9. Verfahren zum Ablösen eines Vorformlings/von Vorformlingen gemäß Anspruch 6, **dadurch gekennzeichnet, dass** es das Vorbeilaufen des unerwünschten Vorformlings voraussieht, um das Einspritzen des unter Druck stehenden Fluidstrahls in den Vorformling auszulösen und das Einspritzen des Fluidstrahls mindestens bis zum vollständigen Vorbeilauf des Greifkopfs (7) an der Spritzdüse (15) des Fluids beizubehalten.
